# EUROPEAN PATENT APPLICATION

(11) **EP 4 825 865 A1**
(43) Date of publication of application: **21.10.2026**
(21) Application number: 26173149.1
(22) Date of filing: 17.04.2026
(51) Int. Cl.: C07K 5/062, A61K 38/00, A61P 17/00, A61P 17/18

(54) **PEPTIDE FOR IMPROVING SKIN CONDITION THROUGH ACTIVATION OF HSP47 AND COSMETIC COMPOSITION CONTAINING THE SAME**

(30) Priority: 18.04.2025 KR 20250050599; 30.03.2026 KR 20260057090
(71) Applicant: Incospharm Corporation, Daejeon 34000 (KR)
(72) Inventor: YOON, Seokjeong, 35210 Daejeon (KR); LEE, JaEun, 34002 Daejeon (KR); KIM, Heungjae, 34010 Daejeon (KR); KIM, Yeonjae, 34306 Daejeon (KR); JEONG, Sekyoo, 30150 Sejong-si (KR); CHUNG, Hwa-Jee, 30150 Sejong-si (KR); PARK, Keedon, 35241 Daejeon (KR)
(74) Representative: HGF

(57) **Abstract**

The present disclosure provides a peptide for improving skin condition, and the peptide of the present disclosure may promote collagen synthesis through an increase in HSP47 expression in skin cells and may provide effects of alleviating wrinkles, enhancing skin elasticity, and improving skin structure through skin-protective effects.

## Description

### TECHNICAL FIELD

The following disclosure relates to a peptide that promotes expression of Heat Shock Protein 47 (HSP47) and thereby improves skin condition, and thus the peptide of the present disclosure may be applied to a cosmetic composition that provides effects of alleviating wrinkles, enhancing skin elasticity, strengthening the skin barrier, and soothing the skin.

### BACKGROUND

The skin, which is composed of the epidermis, dermis, and subcutaneous tissue, is the largest organ of the human body and performs various roles, such as a protective function, a barrier function, a thermoregulatory function, an excretory function, and a respiratory function. However, due to various external environmental factors, the thicknesses of the epidermis, dermis, and subcutaneous tissue decrease, and the composition and content of a lipid barrier, which is responsible for the barrier function of the skin, are changed, such that its function rapidly deteriorates, thereby causing aging. From a molecular biological perspective, skin aging is known to be primarily caused by a decrease in collagen that forms a matrix in the dermis of the skin. Collagen is a substance that accounts for 70 to 80% of the dermis of the skin and is a representative substance involved in maintaining skin elasticity. According to the results of a study published in the Journal of the Society of Cosmetic Scientists of Korea, Vol. 38, No. 4, Issue No. 87, collagen peptides are known to have skin recovery and skin regeneration effects. As aging progresses, collagen production decreases, or degradation thereof is promoted by external factors such as ultraviolet rays, thereby increasing skin wrinkles and accelerating skin aging. In order to solve problems such as skin aging and wrinkles, various studies on collagen have been conducted.

In recent years, peptides have attracted attention as functional materials for preventing skin aging and skin wrinkles. Peptides are materials attracting attention in various research fields, such as cosmetics, foods, and pharmaceuticals, and are polymers in which about 2 to 50 amino acids among 20 types of essential amino acids are linked through amide bonds. Peptides are simpler in size and structure than proteins, but are known to have very strong interactions with proteins in vivo and to play important roles, for example, in metabolic processes in vivo. Peptides are not only substances that are present in vivo per se, but are also materials having excellent safety compared to conventional materials because, even when introduced into the body, they remain active for a certain period of time and are then decomposed into amino acids, and, as constituents of proteins, also help activate skin regeneration. Due to the advantages described above, studies using peptides as substances for improving skin condition are being actively conducted, and demand for peptides exhibiting further improved effects of improving skin condition continues to increase.

Meanwhile, in the biosynthesis process of collagen, Heat Shock Protein 47 (HSP47) is known to act as a collagen-specific molecular chaperone and to play an important role in folding and stabilization of collagen. However, studies on peptides that effectively regulate expression of HSP47 and provide effects of improving skin condition are still insufficient.

### SUMMARY

An embodiment of the present disclosure is directed to providing a peptide for improving skin condition, the peptide having an excellent collagen synthesis effect and exhibiting excellent effects in improving signs of skin aging, such as skin wrinkles, skin sagging, and reduced elasticity, and a cosmetic composition containing the same.

In one general aspect, there is provided a peptide represented by the following Chemical Formula 1.

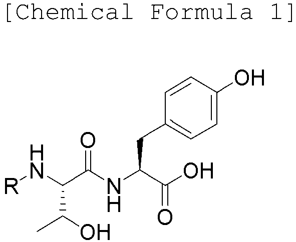

In Chemical Formula 1, R may be hydrogen or C1-C20 alkylcarbonyl.

In Chemical Formula 1, R may be hydrogen.

In Chemical Formula 1, R may be C1-C20 alkylcarbonyl.

In Chemical Formula 1, R may be C1-C10 alkylcarbonyl.

In Chemical Formula 1, R may be hexanoyl, acetyl, or palmitoyl.

In Chemical Formula 1, R may be hexanoyl.

In Chemical Formula 1, R may be acetyl.

In Chemical Formula 1, R may be palmitoyl.

The peptide may be used for improving skin condition.

The peptide may promote expression of Heat Shock Protein 47 (HSP47).

The peptide may promote collagen production.

The collagen production may result from a process including biosynthesis of COL1A1 (Type I pro-collagen) and COL3A1 (Type III pro-collagen).

In another general aspect, there is provided a cosmetic composition containing, as an active ingredient, the peptide according to an exemplary embodiment of the present disclosure.

The cosmetic composition may be in the form of a cosmetic water, a skin toner, a lotion, a nourishing lotion, a nourishing cream, a massage cream, an essence, a pack, or a microneedle patch.

In another general aspect, there is provided a cosmetic composition for improving skin condition containing, as an active ingredient, the peptide according to an exemplary embodiment of the present disclosure.

The cosmetic composition may be in the form of a cosmetic water, a skin toner, a lotion, a nourishing lotion, a nourishing cream, a massage cream, an essence, a pack, or a microneedle patch.

The cosmetic composition may promote expression of Heat Shock Protein 47 (HSP47).

The cosmetic composition may promote collagen production, and the collagen production may result from a process including biosynthesis of COL1A1 (Type I pro-collagen) and COL3A1 (Type III pro-collagen).

The improvement of skin condition may include alleviation of wrinkles, enhancement of skin elasticity, inhibition of skin aging, strengthening of the skin barrier, and alleviation of inflammation.

The cosmetic composition may be used for the alleviation of wrinkles.

The cosmetic composition may be used for the improvement of skin elasticity.

The cosmetic composition may be used for the strengthening of the skin barrier.

The cosmetic composition may be used for the inhibition of skin aging.

In one general aspect, there is provided a peptide according to the present disclosure for use in treating inflammation of the skin. It may be that the peptide is a peptide in which R is acetyl.

Other features and aspects will be apparent from the following detailed description, the drawings, and the claims.

### BRIEF DESCRIPTION OF THE DRAWINGS

FIG. 1 is a view showing results of observing expression of HSP47, COL1A1, and COL3A1 proteins and a control (β-actin) according to a peptide treatment concentration of Example 1.
FIG. 2 is a view quantifying expression levels of HSP47, COL1A1, and COL3A1 proteins according to the peptide treatment concentration of Example 1.
FIG. 3 is a view showing results of observing expression of HSP47 and COL1A1 proteins and a control (β-actin) according to peptide treatment concentrations of Examples 2 to 4.
FIG. 4 is a view quantifying expression levels of HSP47 and COL1A1 proteins according to the peptide treatment concentrations of Examples 2 to 4.
FIG. 5 is a view showing changes in expression of anti-aging-related proteins (Collagen, HSP47, and Collagen-17) caused by Peptide (2) of Example 2 (top: an image showing an expression pattern of collagen observed in the dermis of the skin; middle: an image showing expression of the HSP47 protein; bottom: an image showing expression of the Collagen-17 protein).
FIG. 6 is a view quantifying changes in expression of anti-aging-related proteins (Collagen, HSP47, and Collagen-17) caused by Peptide (2) of Example 2.
FIG. 7 is a view showing changes in expression of skin barrier-related proteins (Involucrin, Loricrin, and SPT) caused by Peptide (2) of Example 2 (top: an image showing expression of Involucrin, which is a skin barrier marker; middle: an image showing expression of Loricrin, which is a skin barrier marker; bottom: an image showing expression of SPT, which is a ceramide synthesis marker).
FIG. 8 is a view quantifying changes in expression of skin barrier-related proteins (Involucrin, Loricrin, and SPT) caused by Peptide (2) of Example 2.
FIG. 9 is a view showing changes in expression of IL-6, which is an inflammation-related marker, caused by Peptide (2) of Example 2.
FIG. 10 is a view quantifying changes in expression of IL-6, which is an inflammation-related marker, caused by Peptide (2) of Example 2.

### DETAILED DESCRIPTION OF EMBODIMENTS

Hereinafter, the present disclosure will be described in detail. Terms used herein should be construed as having meanings generally understood by those skilled in the art unless otherwise defined. The drawings and exemplary embodiments herein are provided so that those skilled in the art may easily understand and practice the present disclosure. Content that may obscure the gist of the present disclosure may be omitted from the drawings and exemplary embodiments, and the present disclosure is not limited to the drawings and exemplary embodiments.

As used herein, singular forms are intended to include plural forms as well, unless the context clearly indicates otherwise.

In addition, a numerical range used herein includes upper and lower limits and all values within the range, increments logically derived from the form and width of the defined range, all values defined by double limitations, and all possible combinations of the upper and lower limits of numerical ranges defined in different forms. Unless otherwise specifically defined in the specification of the present disclosure, values outside the numerical range that may occur due to experimental error or rounding are also included within the defined numerical range.

As used herein, the terms "comprise", "include", "have", and the like indicate the presence of features or components described in the specification, and do not, unless otherwise specifically limited, preclude the possibility that one or more other features or components may be added.

As used herein, "improvement" means any act that alleviates, ameliorates, or beneficially changes a condition.

As used herein, the term "improvement of skin condition" may broadly refer to a process or effect of treating, reducing, or alleviating skin damage caused by intrinsic or extrinsic factors, and may be interpreted as indicating, for example, alleviation of wrinkles, improvement of skin elasticity, wound healing, strengthening of the skin barrier, alleviation of inflammation, or inhibition of skin aging, but is not limited thereto.

In embodiments, the term "improvement of skin condition" in relation to a cosmetic formulation, may refer to the alleviation of wrinkles, improvement of skin elasticity, strengthening of the skin barrier, or inhibition of skin aging.

Herein, "alleviation of wrinkles", "improvement of skin elasticity", and "wound healing" may refer to any action that increases a total amount of extracellular matrix factors, including promotion of collagen synthesis. In addition, "strengthening of the skin barrier" may refer to strengthening an inherent function of the skin that prevents leakage of moisture and nutrients from the skin to the outside and prevents penetration of harmful substances, such as bacteria and viruses, into the skin. In addition, "inhibition of skin aging" may refer to inhibiting deterioration of skin functions, such as wrinkles, skin sagging, and reduction in elasticity. In this case, the skin aging may be photoaging, for example, skin aging caused by ultraviolet rays.

In the present disclosure, Heat Shock Protein 47 (HSP47) is a type of Heat Shock Protein (HSP) induced by Heat Shock, and is a collagen-specific molecular chaperone that is essential for intracellular transport and molecular maturation common to the collagen synthesis process. Specifically, HSP47 may specifically recognize and bind arginine (Arg) at the Yaa position in a Gly-Xaa-Arg repeating sequence of collagen, thereby preventing local unwinding and stabilizing a thermodynamically unstable collagen structure to regulate biosynthesis of collagen.

Specifically, the present disclosure provides a peptide represented by the following Chemical Formula 1.

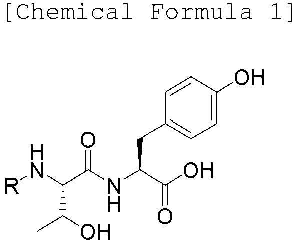

In Chemical Formula 1, R may be hydrogen or C1-C20 alkylcarbonyl.

Conventional methods for delivering substances such as collagen using cell-penetrating peptides and liposomes involve risks of intracellular toxicity or re-release, whereas the peptide of the present disclosure is a peptide for improving skin condition and may overcome such limitations and directly target HSP47, such that HSP47 acts as a collagen-specific molecular chaperone to regulate folding and stabilization of collagen and, through such action, may affect collagen biosynthesis and secretion processes.

Collagen is a major constituent of the dermis of the skin, and is known to account for about 70 to 80% of a dry weight of the dermis and about 20 to 30% of a volume of the dermis. Collagen is responsible for functions of supporting tissue binding force and cell adhesion and maintaining cell division and differentiation during wound healing. The peptide of the present disclosure may promote synthesis of collagen in vivo and may increase matrix components of the dermis, and thus effects such as improved wound healing and increased elasticity may be expected.

In addition, the peptide of the present disclosure may provide effects of alleviating wrinkles, enhancing skin elasticity, and improving skin structure through skin-protective effects resulting from collagen synthesis, and specifically, the peptide of the present disclosure may be used for alleviating skin wrinkles, inhibiting skin aging, strengthening the skin barrier, and alleviating inflammation, but is not limited thereto.

The peptide according to the present disclosure may be prepared using any conventional peptide synthesis method known in the art without limitation. For example, the peptide may be synthesized by a chemical synthesis method, such as a solution phase peptide synthesis method, a solid-phase peptide synthesis method, a fragment condensation method, or a chemical synthesis method using F-moc or T-boc chemistry; and a biological synthesis method of expressing, in genetically engineered *Escherichia coli,* a polynucleotide encoding a fusion peptide for improving skin condition, and specifically may be synthesized by a solid-phase peptide synthesis method, but is not limited thereto.

According to an exemplary embodiment, in Chemical Formula 1, R may be hydrogen.

According to an exemplary embodiment, in Chemical Formula 1, R may be C1-C20 alkylcarbonyl.

According to an exemplary embodiment, in Chemical Formula 1, R may be C1-C10 alkylcarbonyl.

According to an exemplary embodiment, in Chemical Formula 1, R may be hexanoyl, acetyl, or palmitoyl, and specifically may be acetyl.

According to an exemplary embodiment, in Chemical Formula 1, R may be acetyl.

According to an exemplary embodiment, in Chemical Formula 1, R may be hexanoyl.

According to an exemplary embodiment, in Chemical Formula 1, R may be palmitoyl.

Since the peptide has a much lower molecular weight than other proteins and thus exhibits excellent skin permeability, the peptide may effectively improve skin condition even when topically applied to the skin. That is, the peptide may be used as an active ingredient in a cosmetic composition for improving skin condition.

According to an exemplary embodiment, the peptide may promote expression of Heat Shock Protein 47 (HSP47).

According to an exemplary embodiment, the peptide may promote collagen production.

The collagen production may result from a process including biosynthesis of COL1A1 (Type I pro-collagen) and COL3A1 (Type III pro-collagen).

The present disclosure provides a cosmetic composition for improving skin condition containing, as an active ingredient, the peptide according to an exemplary embodiment.

According to an exemplary embodiment, the cosmetic composition for improving skin condition of the present disclosure may contain a cosmetically effective amount of the above-described peptide and a cosmetically acceptable carrier. The term "cosmetically effective amount" means an amount sufficient to achieve the efficacy of improving skin condition of the composition of the present disclosure.

The cosmetic composition may contain 0.001 to 0.050 wt% of the peptide of the present disclosure based on the total weight of the cosmetic composition. Specifically, the lower limit may be 0.001, 0.005, 0.010, 0.015, 0.020, 0.025, or 0.030 wt%, and the upper limit may be 0.050, 0.045, 0.040, or 0.035 wt%, but the present disclosure is not limited thereto.

The cosmetic composition may be formulated in any form commonly prepared in the art, and may be, for example, in the form of a cosmetic water, a skin toner, a lotion, a nourishing lotion, a nourishing cream, a massage cream, an essence, a pack, or a microneedle patch.

When the form of the cosmetic composition is a paste, a cream, or a gel, one or two or more selected from animal oil, vegetable oil, wax, paraffin, starch, tragacanth, a cellulose derivative, polyethylene glycol, silicone, bentonite, silica, talc, and zinc oxide may be further used as a carrier component.

When the form of the cosmetic composition is a powder or a spray, lactose, talc, silica, aluminum hydroxide, calcium silicate, or polyamide powder may be further used as a carrier component, and particularly when the form is a spray, the cosmetic composition may additionally contain a propellant such as chlorofluorohydrocarbon, propane/butane, or dimethyl ether.

When the form of the cosmetic composition is a solution or an emulsion, a solvent, a solubilizing agent, or an emulsifying agent may be further used as a carrier component, and examples thereof include water, ethanol, isopropanol, ethyl carbonate, ethyl acetate, benzyl alcohol, benzyl benzoate, propylene glycol, 1,3-butylene glycol, a glycerol aliphatic ester, polyethylene glycol, or a fatty acid ester of sorbitan.

When the form of the cosmetic composition is a suspension, a liquid diluent such as water, ethanol, or propylene glycol; or a suspending agent such as ethoxylated isostearyl alcohol, a polyoxyethylene sorbitol ester, or a polyoxyethylene sorbitan ester may be further used as a carrier component, and crystalline cellulose, aluminum metahydroxide, bentonite, agar, or tragacanth may also be additionally used.

When the form of the cosmetic composition is a surfactant-containing cleanser, an aliphatic alcohol sulfate, an aliphatic alcohol ether sulfate, a sulfosuccinic acid monoester, an isethionate, an imidazolinium derivative, a methyl taurate, a sarcosinate, a fatty acid amide ether sulfate, an alkylamidobetaine, an aliphatic alcohol, a fatty acid glyceride, a fatty acid diethanolamide, vegetable oil, a lanolin derivative, or an ethoxylated glycerol fatty acid ester may be used as a carrier component.

According to an exemplary embodiment, the cosmetic composition may further contain additional additives commonly used in cosmetic compositions depending on an intended purpose, and may contain, for example, one or two or more aqueous additives selected from a stabilizer, an emulsifier, a thickener, a moisturizer, a liquid crystal membrane strengthening agent, a pH adjuster, a water-soluble polymer, a film-forming agent, a metal ion sequestering agent, an organic amine, a polymer emulsion, a skin nutrient, an antioxidant, an antioxidant aid, a preservative, and a fragrance; and one or two or more oil-based additives selected from fats and oils, waxes, a hydrocarbon oil, a higher fatty acid oil, a higher alcohol, a synthetic ester oil, and a silicone oil.

According to an exemplary embodiment, the cosmetic composition may contain two or more different types of peptides selected from the peptides described above, and may exhibit a further improved effect on skin condition due to a synergistic effect, as compared with a case in which only one type is contained.

The microneedle patch may include a microneedle part in which a plurality of microneedles containing the peptide are positioned; and a base part coupled to the microneedle part.

Hereinafter, the peptide for improving skin condition according to the present disclosure and a cosmetic composition containing the same will be described in further detail through specific examples. However, the following examples are only a reference to explain the present disclosure in detail, and the present disclosure is not limited thereto and may be implemented in various forms. In addition, the terms used in the description of the present disclosure are merely used to effectively describe specific examples, but are not intended to limit the present disclosure.

### [Example 1] Preparation of Peptide (1)

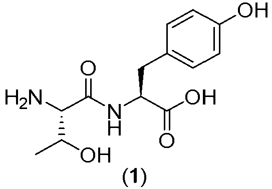

In Example 1, synthesis of the peptide was performed according to Fmoc Solid Phase Peptide Synthesis using 9-fluorenylmethoxycarbonyl (Fmoc) as a protecting group for an Nα-amino acid, and the peptide was elongated using an N-hydroxybenzotriazole-diisopropylcarbodiimide (HOBt-DIC) coupling agent (Wang C. Chan, Perter D. white, "Fmoc solid phase peptide synthesis" Oxford).

### 1-1. Synthesis of Compound 1a

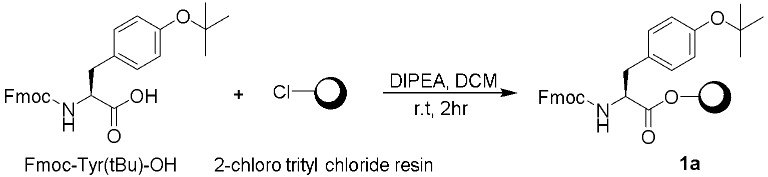

A 2-chloro trityl chloride resin (208 mg, 1 equivalent) was placed in a 10 ml reaction vessel, and N-Fmoc-O-(tert-butyl)-L-tyrosine (Fmoc-Tyr(tBu)-OH) (275.7 mg, 2 equivalents) and N,N-diisopropylethylamine (DIPEA) (315 µl, 6 equivalents) were dissolved in 6 ml of dichloromethane (DCM) and then added thereto, and the reaction was allowed to proceed at room temperature for 2 hours. The reaction solution was removed by filtration, and the synthesized resin was sequentially washed with 20 ml of each of DCM, MeOH, DCM, and N,N-dimethylformamide (DMF). After vacuum drying, Compound 1a (Fmoc-Tyr(tBu)-O-2-chloro trityl resin) in a solid-phase form was obtained in a quantitative yield.

### 1-2. Synthesis of Compound 1b

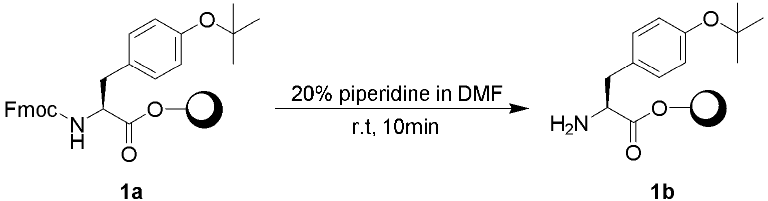

Compound 1a and 6 ml of 20% piperidine in DMF were placed in a 10 ml reaction vessel and reacted at room temperature for 10 minutes, and then the reaction solution was removed by filtration. The reaction solution was removed by filtration, and the synthesized resin was sequentially washed with 20 ml of each of DCM, MeOH, DCM, and DMF. After vacuum drying, Compound 1b (H-Tyr(tBu)-O-2-chloro trityl resin) in a solid-phase form was obtained in a quantitative yield.

### 1-3. Synthesis of Compound 1c

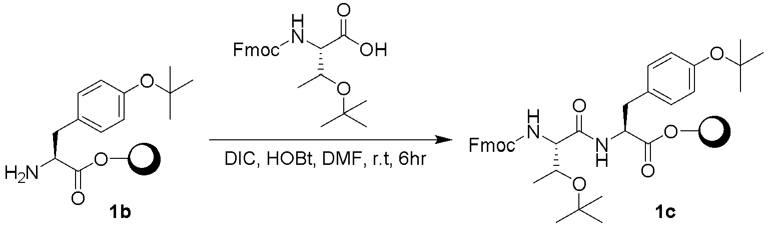

Compound 1b, N-Fmoc-O-(tert-butyl)-L-threonine (Fmoc-Thr(tBu)-OH) (477 mg, 4 equivalents), 1-hydroxybenzotriazole (HOBt) (162 mg, 4 equivalents), and N,N'-diisopropylcarbodiimide (DIC) (186 µl, 4 equivalents) were dissolved in 60 ml of DMF and added to a 10 ml reaction vessel, and the reaction was allowed to proceed at room temperature for 6 hours. The reaction solution was removed by filtration, and the synthesized resin was sequentially washed with 200 ml of each of DCM, MeOH, DCM, and DMF. After vacuum drying, Compound 1c (Fmoc-Thr(tBu)-Tyr(tBu)-O-2-chloro trityl resin) in a solid-phase form was obtained in a quantitative yield.

### 1-4. Synthesis of Compound 1d

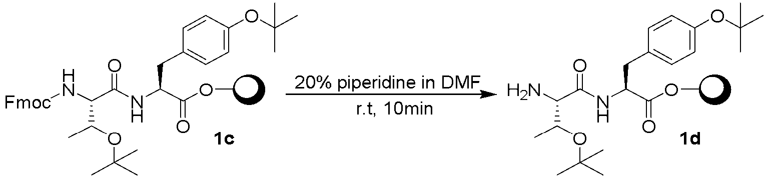

Compound 1c and 6 ml of 20% piperidine in DMF were placed in a 10 ml reaction vessel and reacted at room temperature for 10 minutes. The reaction solution was removed by filtration, and the synthesized resin was sequentially washed with 20 ml of each of DCM, MeOH, DCM, and DMF. After vacuum drying, Compound 1d (H-Thr(tBu)-Tyr(tBu)-O-2-chloro trityl resin) in a solid-phase form was obtained in a quantitative yield.

### 1-5. Synthesis of Peptide 1

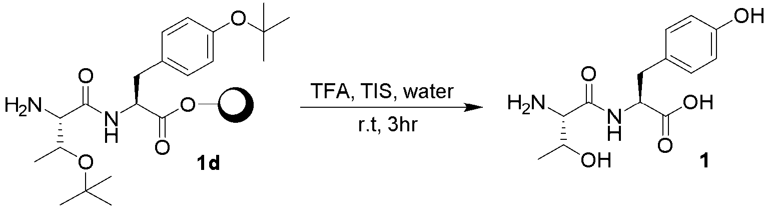

Compound 1d and 6 ml of a cleavage cocktail (trifluoroacetic acid (TFA):triisopropylsilane (TIS):distilled water (DW) = 95:2.5:2.5; v:v:v) were added to a 10 ml reaction vessel and reacted at room temperature for 3 hours. The reaction solution was collected by filtration, and 40 ml of diethyl ether was added thereto to precipitate a product. The solid product was collected using a centrifuge and washed twice with 40 ml of diethyl ether. The obtained solid product was purified using preparative high-performance liquid chromatography (Prep-HPLC, column C18, 10 µm, 250 mm × 22 mm), freeze-dried, and Peptide (1) (molecular weight: 282.29) was obtained in an amount of 59 mg at a yield of 70%.

### [Example 2] Preparation of Peptide (2)

The desired Peptide (2) was prepared by acetylating the N-terminus of Peptide (1) of Example 1.

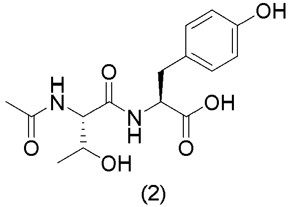

### 2-1. Synthesis of Compound 2a

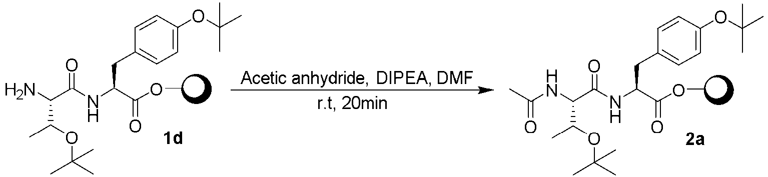

Compound 1d was placed in a 10 ml reaction vessel, and acetic anhydride (283 µl, 10 equivalents) and DIPEA (522 µl, 10 equivalents) were dissolved in 10 ml of DMF and added thereto, and the reaction was allowed to proceed at room temperature for 20 minutes. The reaction solution was removed by filtration, and the synthesized resin was sequentially washed with 20 ml of each of DCM, MeOH, DCM, and DMF. After vacuum drying, Compound 2a (Ac-Thr(tBu)-Tyr(tBu)-O-2-chloro trityl resin) in a solid-phase form was obtained in a quantitative yield.

### 2-2. Synthesis of Peptide (2)

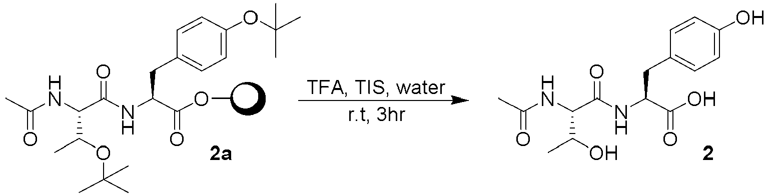

Compound 2a and 6 ml of a cleavage cocktail (TFA:TIS:DW = 95:2.5:2.5; v:v:v) were added to a 10 ml reaction vessel and reacted at room temperature for 3 hours. The reaction solution was collected by filtration, and 40 ml of diethyl ether was added thereto to precipitate a product. The solid product was collected using a centrifuge and washed twice with 40 ml of diethyl ether. The obtained solid product was purified using preparative high-performance liquid chromatography (Prep-HPLC, column C18, 10 µm, 250 mm × 22 mm), freeze-dried, and Peptide (2) (molecular weight: 324.33) was obtained in an amount of 63 mg at a yield of 65%.

### [Example 3] Preparation of Peptide (3)

The desired Peptide (3) was prepared by hexanoylating the N-terminus of Peptide (1) of Example 1.

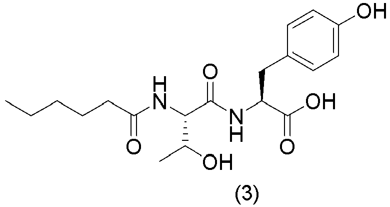

### 3-1. Synthesis of Compound 3a

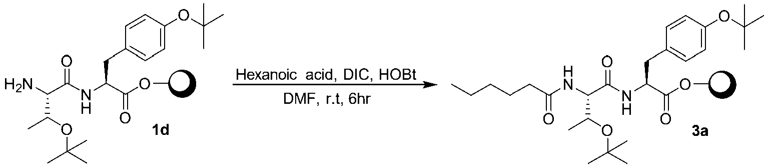

Compound 1d was placed in a 10 ml reaction vessel, and hexanoic acid (140 mg, 4 equivalents), HOBt (162 mg, 4 equivalents), and DIC (186 µl, 4 equivalents) were dissolved in 60 ml of DMF and added thereto, and the reaction was allowed to proceed at room temperature for 6 hours. The reaction solution was removed by filtration, and the synthesized resin was sequentially washed with 200 ml of each of DCM, MeOH, DCM, and DMF. After vacuum drying, Compound 3a (Hexanoyl-Thr(tBu)-Tyr(tBu)-O-2-chloro trityl resin) in a solid-phase form was obtained in a quantitative yield.

### 3-2. Synthesis of Peptide (3)

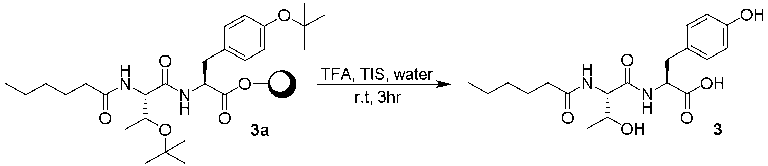

Compound 3a and 6 ml of a cleavage cocktail (TFA:TIS:DW = 95:2.5:2.5; v:v:v) were added to a 10 ml reaction vessel and reacted at room temperature for 3 hours. The reaction solution was collected by filtration, and 40 ml of diethyl ether was added thereto to precipitate a product. The solid product was collected using a centrifuge and washed twice with 40 ml of diethyl ether. The obtained solid product was purified using preparative high-performance liquid chromatography (Prep-HPLC, column C18, 10 µm, 250 mm × 22 mm), freeze-dried, and Peptide (3) (molecular weight: 380.44) was obtained in an amount of 76 mg at a yield of 67%.

### [Example 4] Preparation of Peptide (4)

The desired Peptide (4) was prepared by palmitoylating the N-terminus of Peptide (1) of Example 1.

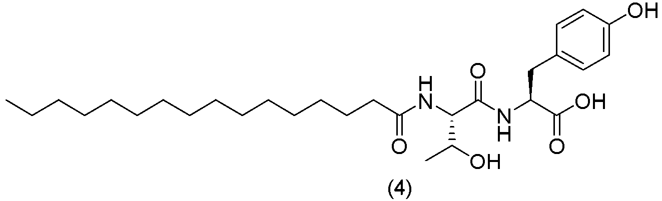

### 4-1. Synthesis of Compound 4a

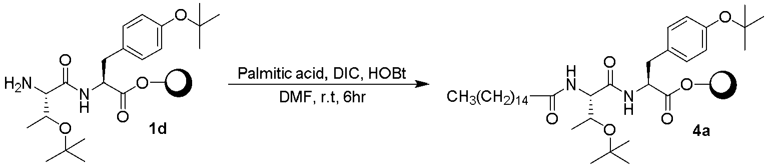

Compound 1d was placed in a 10 ml reaction vessel, and palmitic acid (308 mg, 4 equivalents), HOBt (162 mg, 4 equivalents), and DIC (186 µl, 4 equivalents) were dissolved in 60 ml of DMF and added thereto, and the reaction was allowed to proceed at room temperature for 6 hours. The reaction solution was removed by filtration, and the synthesized resin was sequentially washed with 200 ml of each of DCM, MeOH, DCM, and DMF. After vacuum drying, Compound 4a (Palmitoyl-Thr(tBu)-Tyr(tBu)-O-2-chloro trityl resin) in a solid-phase form was obtained in a quantitative yield.

### 4-2. Synthesis of Peptide (4)

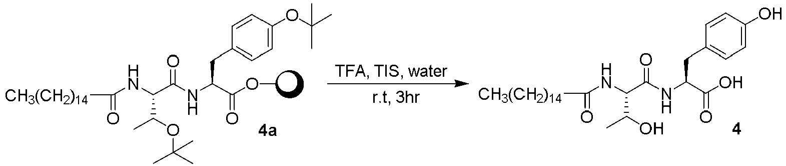

Compound 4a and 6 ml of a cleavage cocktail (TFA:TIS:DW = 95:2.5:2.5; v:v:v) were added to a 10 ml reaction vessel and reacted at room temperature for 3 hours. The reaction solution was collected by filtration, and 40 ml of diethyl ether was added thereto to precipitate a product. The solid product was collected using a centrifuge and washed twice with 40 ml of diethyl ether. The obtained solid product was purified using preparative high-performance liquid chromatography (Prep-HPLC, column C18, 10 µm, 250 mm × 22 mm), freeze-dried, and Peptide (4) (molecular weight: 520.7) was obtained in an amount of 111 mg at a yield of 71%.

### [Evaluation Example 1] Evaluation of Expression Levels of HSP47 Signaling-Related Proteins

Using DMEM culture medium containing 10% FBS, human dermal fibroblasts (HDFs), which are human fibroblasts, were uniformly seeded in a 6-well plate at a density of 1 × 10⁵ cells/well, and then cultured in an incubator at 37°C under 5% CO₂ conditions for 24 hours. Peptide (1) of Example 1 was dissolved in water at a concentration of 100 mM to prepare a concentrated solution, and the concentrated solution was added to each well so that the concentration of the peptide was 10 µM or 100 µM, and the cells were cultured for 24 hours.

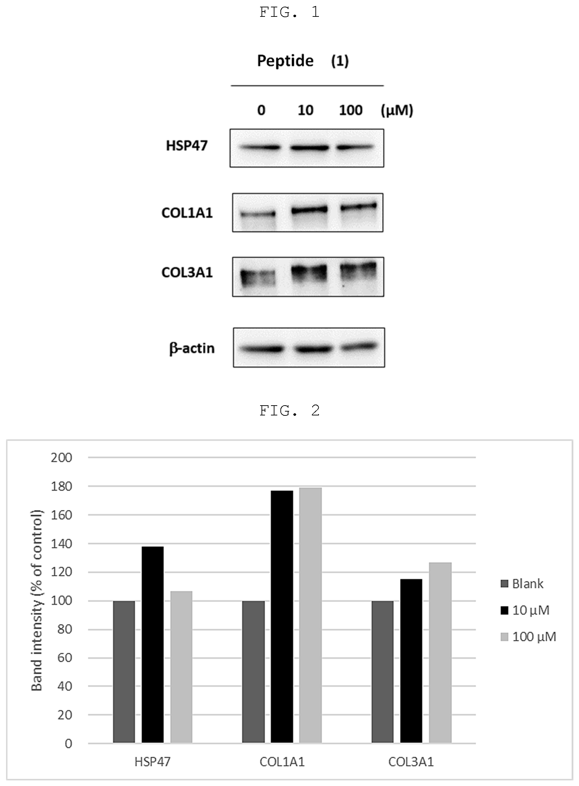

After completion of the culture, the medium was removed, the cells were lysed with a sodium dodecyl sulfate (SDS) sample buffer, and then the proteins were separated by SDS-PAGE gel electrophoresis. Thereafter, the proteins were transferred onto a nitrocellulose (NC) membrane, non-specific binding was blocked using a blocking buffer, antibodies against HSP47, COL1A1, and COL3A1 proteins and HRP-conjugated secondary antibodies against the antibodies (anti-rabbit IgG HRP, Sigma) were allowed to react, an enhanced chemiluminescence (ECL) reaction was performed using an ECL kit (Amersham pharmacia), and ChemiDoc (UVITEC) analysis was performed. The results are shown in FIGS. 1 and 2. β-actin was used as a control for protein amount.

According to FIGS. 1 and 2, it can be seen that production of proteins related to HSP47 signaling is increased by Peptide (1) prepared in Example 1.

### [Evaluation Example 2]

Using DMEM culture medium containing 10% FBS, human dermal fibroblasts (HDFs), which are human fibroblasts, were uniformly seeded in a 6-well plate at a density of 1 × 10⁵ cells/well, and then cultured in an incubator at 37°C under 5% CO₂ conditions for 24 hours. Peptides (2) to (4) prepared in Examples 2 to 4 were each dissolved in DMSO at a concentration of 100 mM to prepare concentrated solutions, and the concentrated solutions were added to each well so that the concentrations of Peptides (2) and (3) prepared in Examples 2 and 3 were 10 µM or 100 µM and the concentration of Peptide (4) prepared in Example 4 was 1 µM or 10 µM, and the cells were cultured for 24 hours.

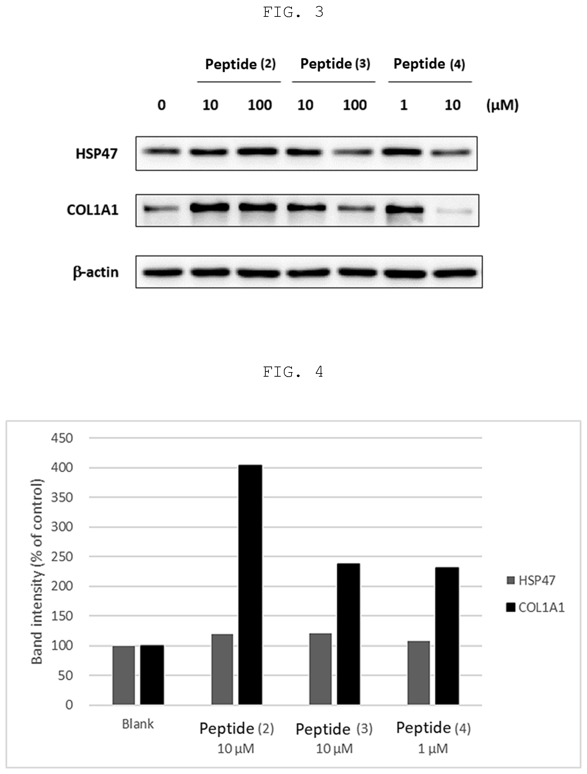

After completion of the culture, the medium was removed, the cells were lysed with an SDS sample buffer, and the proteins were separated by SDS-PAGE gel electrophoresis. Thereafter, the proteins were transferred onto a nitrocellulose (NC) membrane, non-specific binding was blocked using a blocking buffer, antibodies against HSP47 and COL1A1 proteins and HRP-conjugated secondary antibodies against the antibodies (anti-rabbit IgG HRP, Sigma) were allowed to react, an enhanced chemiluminescence (ECL) reaction was performed using an ECL kit (Amersham pharmacia), and ChemiDoc (UVITEC) analysis was performed. The results are shown in FIGS. 3 and 4. β-actin was used as a control for protein amount.

According to FIGS. 3 and 4, it can be seen that production of HSP47 and COL1A1, which is a protein related to HSP47 signaling, is increased by Peptides (2) to (4) prepared in Examples 2 to 4. In particular, in the case of Peptide (2) of Example 2, it can be confirmed that activation levels of HSP47 and COL1A1 proteins are remarkably high.

### [Evaluation Example 3] Evaluation of Anti-Aging Effects

Peptide (2) prepared in Example 2 was applied in an amount of 0.1 mg to the surface of artificial skin tissue (NativeSkin access^{®}, Genoskin), and cultured for 48 hours. After 48 hours, the artificial skin tissue was washed, placed in a 10% formalin solution, and fixed at room temperature for 48 hours. Thereafter, the artificial skin tissue was washed with running distilled water and dehydrated, and embedded in paraffin to prepare a paraffin block.

The prepared paraffin block was sectioned to a thickness of 5 µm and used for analysis of biomarker expression. Immunohistochemical staining was performed using antibodies against HSP47 (Abcam, ab109117) and collagen-17 (Abcam, ab184996), which are used as biomarkers for confirming expression of anti-aging-related proteins in the epidermis of the skin, and images were acquired under a microscope. In addition, staining was performed using Masson's trichrome staining, which is a special staining technique capable of confirming an expression pattern of collagen observed in the dermis of the skin, and images were acquired under a microscope and used for analysis.

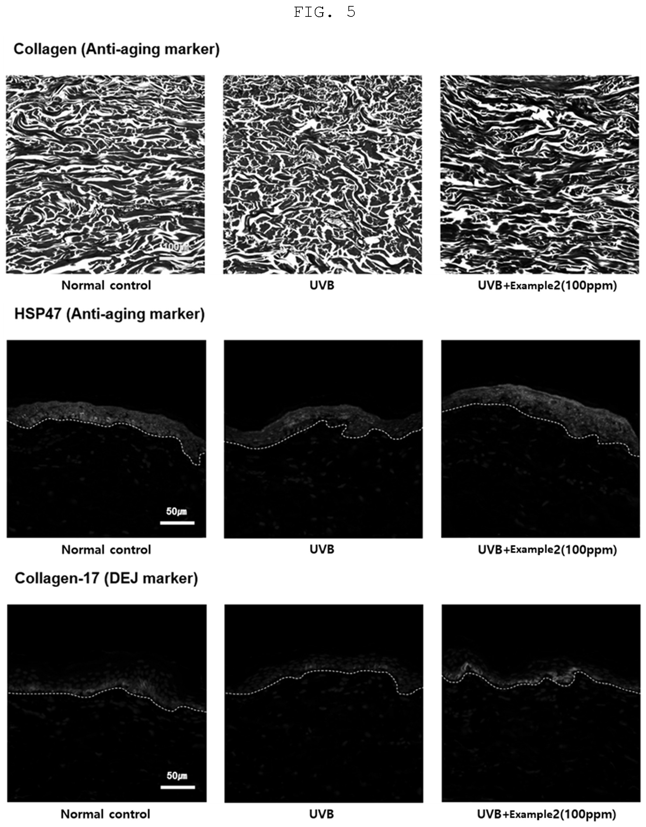

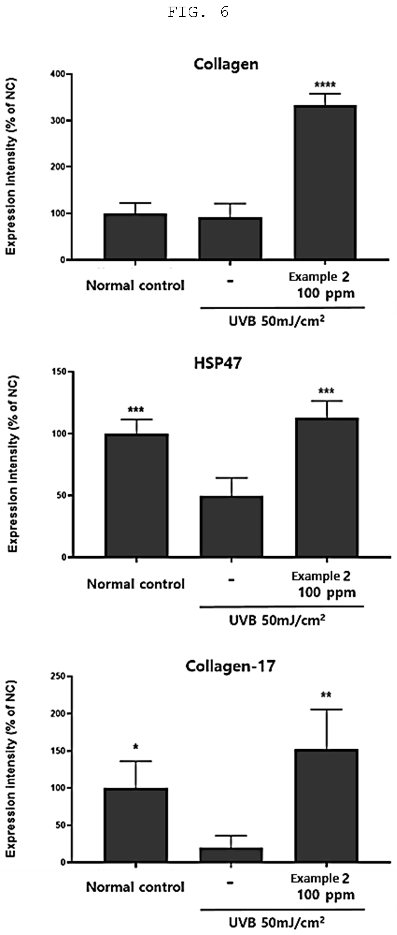

The acquired images were analyzed using the ImageJ (NIH, USA) program. The results are shown in FIGS. 5 and 6.

According to FIGS. 5 and 6, it can be seen that the expression levels of anti-aging-related proteins, Collagen, HSP47, and Collagen-17, which were decreased after ultraviolet irradiation, are increased by Peptide (2) prepared in Example 2.

### [Evaluation Example 4] Evaluation of Skin Barrier Recovery

The paraffin block prepared in the same manner as in Evaluation Example 3 was sectioned to a thickness of 5 µm and used for analysis of biomarker expression.

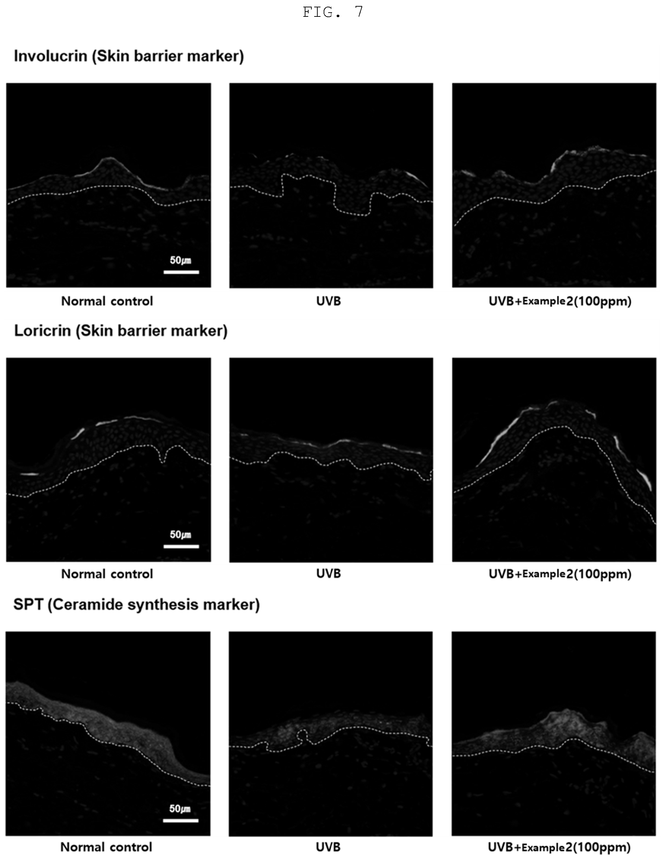

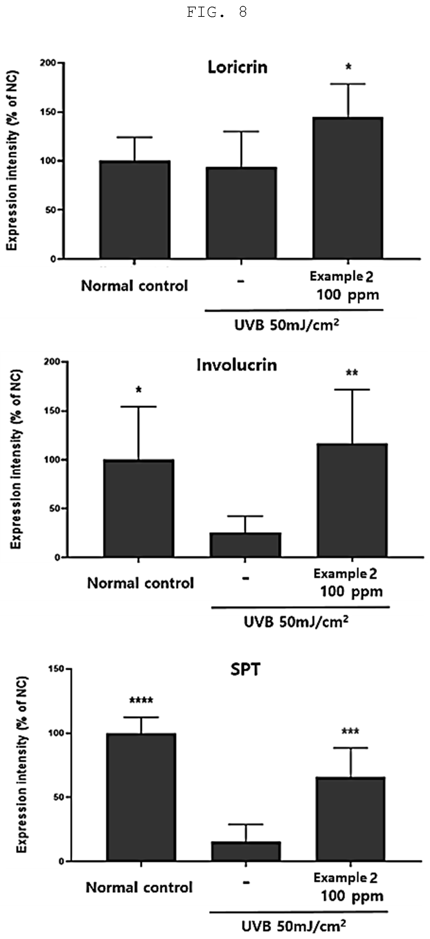

Immunohistochemical staining was performed using antibodies against Involucrin (Abcam, ab53112), Loricrin (Abcam, ab176322), and serine-palmitoyltransferase (SPT, Abcam, ab236900), which are used as biomarkers for confirming expression of skin barrier-related proteins in the epidermis of the skin, and images were acquired under a microscope. The acquired images were analyzed using the ImageJ (NIH, USA) program. The results are shown in FIGS. 7 and 8.

According to FIGS. 7 and 8, it can be seen that the expression levels of skin barrier-related proteins, Involucrin and SPT, which were decreased after ultraviolet irradiation, are increased by Peptide (2) prepared in Example 2. In addition, it can be seen that the expression level of Loricrin, which showed no significant difference before and after ultraviolet irradiation, is also increased by Peptide (2) prepared in Example 2. That is, it can be seen that Peptide (2) of Example 2 provides an effect of strengthening the skin barrier.

### [Evaluation Example 5] Evaluation of Skin-Soothing Effect

The paraffin block prepared in the same manner as in Evaluation Example 3 was sectioned to a thickness of 5 µm and used for analysis of biomarker expression.

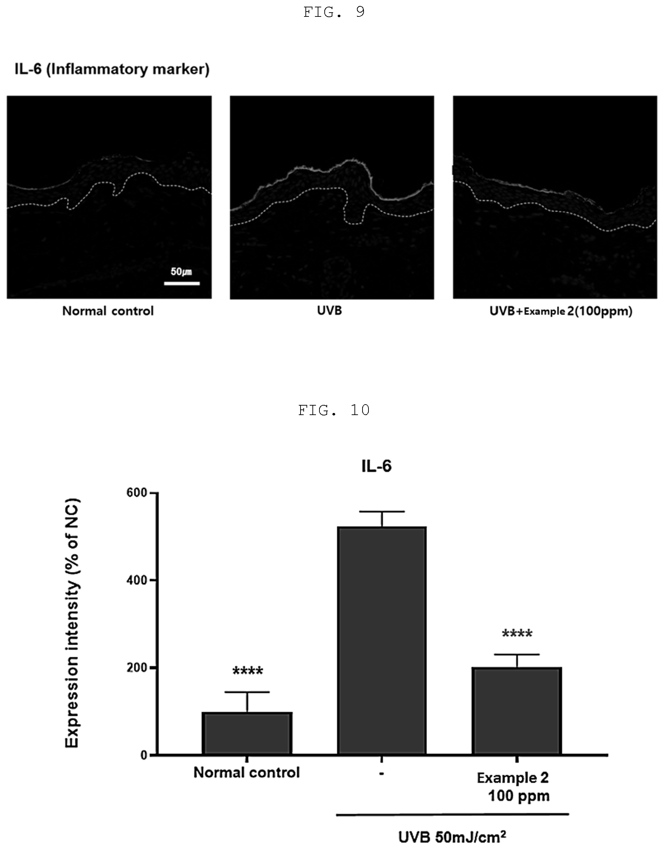

Immunohistochemical staining was performed using an antibody against interleukin-6 (IL-6, Abcam, ab6672), which is used as a biomarker for confirming expression of a skin soothing-related protein in the skin, and images were acquired under a microscope. The acquired images were analyzed using the ImageJ (NIH, USA) program. The results are shown in FIGS. 9 and 10.

According to FIGS. 9 and 10, it can be seen that the expression level of IL-6, an inflammatory cytokine, which was increased after ultraviolet irradiation, is decreased by Peptide (2) prepared in Example 2. That is, it can be seen that Peptide (2) of Example 2 provides a skin-soothing effect.

### [Example 5] Preparation of Cosmetic Composition for Improving Skin Condition (Nourishing Cream)

A nourishing cream containing one or more peptides selected from the peptides prepared in Examples 1 to 4 was prepared by a conventional method according to the composition shown in Table 1.

**[Table 1]**

| | Content (wt%) |
|---|---|
| Peptide for improving skin condition | 0.03 |
| Glycerin | 3.00 |
| Sodium hyaluronate | 0.50 |
| Sodium ascorbyl phosphate | 1.00 |
| Tocopheryl acetate | 0.50 |
| 1,3-Butylene glycol | 5.00 |
| D-Panthenol | 0.10 |
| Cetyl alcohol | 2.00 |
| Stearyl alcohol | 1.50 |
| Glyceryl stearate | 2.00 |
| Polysorbate 60 | 1.50 |
| Caprylic/capric triglyceride | 0.30 |
| Phenoxyethanol | 0.80 |
| Disodium EDTA | 0.10 |
| Purified water | Balance (total 100) |

As set forth above, the peptide for improving skin condition according to an exemplary embodiment of the present disclosure may promote collagen synthesis through an increase in HSP47 expression in skin cells and may provide effects of alleviating wrinkles, enhancing skin elasticity, and improving skin structure through skin-protective effects.

Hereinabove, although the present disclosure has been described with reference to specific matters and limited examples and comparative examples, they have been provided only for assisting in a general understanding of the present disclosure. Therefore, the present disclosure is not limited to the examples, and various modifications and changes may be made by those skilled in the art to which the present disclosure pertains from this description.

Accordingly, the spirit of the present disclosure should not be construed as being limited to the described exemplary embodiments, and the claims and all modifications equal or equivalent thereto are intended to fall within the spirit of the present disclosure.

## Claims

1. A peptide represented by the following Chemical Formula 1: 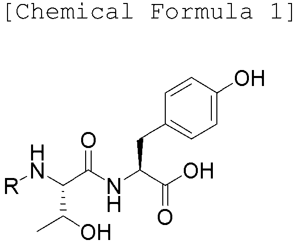 wherein R is hydrogen or C1-C20 alkylcarbonyl.

2. The peptide of claim 1, wherein R is hydrogen.

3. The peptide of claim 1, wherein R is hexanoyl, acetyl, or palmitoyl.

4. The peptide of any preceding claim, wherein the peptide is used for improving skin condition.

5. The peptide of claim 4, wherein the peptide promotes expression of Heat Shock Protein 47 (HSP47).

6. The peptide of claim 5, wherein the peptide promotes collagen production.

7. The peptide of claim 6, wherein the collagen production results from a process including biosynthesis of COL1A1 (Type I pro-collagen) and COL3A1 (Type III pro-collagen).

8. A cosmetic composition comprising the peptide of any one of claims 1 to 7.

9. The cosmetic composition of claim 8 for improving skin condition.

10. The cosmetic composition of claim 8 or claim 9, wherein the cosmetic composition is in the form of a cosmetic water, a skin toner, a lotion, a nourishing lotion, a nourishing cream, a massage cream, an essence, a pack, or a microneedle patch.

11. The cosmetic composition of any one of claims 8 to 10, wherein the cosmetic composition promotes expression of Heat Shock Protein 47 (HSP47).

12. The cosmetic composition of claim 11, wherein the cosmetic composition promotes collagen production.

13. The cosmetic composition of claim 12, wherein the collagen production results from a process including biosynthesis of COL1A1 (Type I pro-collagen) and COL3A1 (Type III pro-collagen).

14. The cosmetic composition of claim 9 or claim 10, wherein the improvement of skin condition includes alleviation of wrinkles, enhancement of skin elasticity, inhibition of skin aging, strengthening of the skin barrier, and alleviation of inflammation.

15. The peptide of any one of claims 1 to 3, for use in treating inflammation of the skin;
optionally wherein R is acetyl.
